# EUROPEAN PATENT APPLICATION

(11) **EP 4 741 488 A1**
(43) Date of publication of application: **13.05.2026**
(21) Application number: 24211767.9
(22) Date of filing: 08.11.2024
(51) Int. Cl.: C12M 3/00, C12M 1/24, C12M 1/00, C12M 1/04, C12M 1/12

(54) **PROCESS FOR THE PRODUCTION OF FOODSTUFFS FROM CULTURED CELLS**

(71) Applicant: Bühler AG, 9240 Uzwil (CH)
(72) Inventor: Witschi, Friedrich, 8260 Stein am Rhein (CH); Buchmann, Leandro, 8408 Winterthur (CH); Greive, Gero, 9535 Wilen b. Wil (CH)
(74) Representative: Hepp Wenger Ryffel AG

(57) **Abstract**

The present invention is related to a process for cultivating cells or microorganisms, comprising the steps of providing a three-dimensional flexible membrane structure which is permeable to gases and impermeable to liquid and solid components, filling the membrane structure with a composition containing cells or microorganisms, and treating the filled membrane structure under cultivation conditions which allow the cells or microorganisms to grow, characterized in that the membrane structure has a longest dimension of 0.1 cm to 40 cm, preferably 1 to 20 cm. The present invention is furthermore related to a process for the preparation of a food product, comprising the steps of cultivating cells or microorganisms in a process described above, processing the product thus obtained into the food product, characterized in that the composition containing cells or microorganisms has food quality.

## Description

The present invention relates to a process for the production of foodstuffs from cultured cells.

Innovative food products, such as products that imitate properties of meat, poultry, fish, seafood, or products derived therefrom, that are based on vegetable proteins are becoming increasingly important as part of the sustainability trend.

A major challenge for the acceptance of such products is to match their texture, their nutrition quality, their color and their organoleptic properties (bite) as far as possible to the corresponding properties of real meat, poultry, or fish and seafood products. However, products produced using the known methods and methods available on the market differ significantly from real meat in their texture, their nutrition quality, their color and their bite. This applies to the fibrousness as well as the tenderness or juiciness. In general, each innovative food product faces the same challenges as to texture, nutrition quality, color and bite, in order to ensure consumer acceptance.

A typical product that imitates properties of meat, poultry, fish, seafood, or products derived therefrom, is made using a vegetable protein by a method wherein the raw materials (including the vegetable (plant-based) protein) are weighed/dosed and mixed, optionally preconditioned, and subsequently processed in an extruder. Preferably, the thus obtained product is led out of the extruder through a cooling die and cut.

This is a very high-energy process in which thermal energy (heating the extruder housing and cooling water in a cooling die or introduction of steam into the extruder) and mechanical energy (specific mechanical energy input) are introduced. However, an increased supply of energy in the process inevitably leads to a stronger texturing, which in turn has a negative effect on the bite or the tenderness of the end product - the products have a rubber-like character.

In WO 2021/032866 A1, a method was described which results in the production of a foamed product that has improved characteristics over known food products that imitate properties of meat and fish.

It also known in the art to use cultured meat as alternative meat product. Cultured meat, also called synthetic meat, cell-cultured meat, clean meat, and in vitro meat, is meat grown in cell culture instead of inside animal (e.g. Verbeke et al., Journal of Integrative Agriculture 14.2 (2015): 285-294). For example, muscle cells are grown ex vivo attached to either a 2D or 3D support structure. However, the currently available cultured meat products have not yet gained wide acceptance, since they also exhibit the problems of texture, color and bite discussed above.

The current industrial processes for culturing cells or microorganisms are burdensome and expensive. The typically used bioreactors are rigid containers of stainless steel that have to be lavishly sterilized, and the typically fermentation processes only provide low yields.

In WO 2005/049784 A1, a cell culture apparatus was suggested for obtaining higher yields. Said a cell culture apparatus comprises a culture chamber, which is typically a bag having a length of about 3 m and a width of about 0,3-0.9 m, in order to enable cultivation of a large volume of a cellular material. In order to provide the interior of such a bag with a sufficient amount of oxygen, the culture chamber is provided for cultivation on a wave induction mechanism. The culture chamber is not completely filled and is moved such that waves protrude through the culture chamber. This system is not optimal, since it requires continuous movement of the culture chamber. This may prevent cells from adhering to surface areas, if such movement is too vigorous, and thus impedes growth to a cellular product that resembles a natural product. Moreover, the required patterns of movement are difficult to scale and consume energy, and thus any necessity for movements is detrimental per se.

In WO 2024/211384 A1, an edible microcarrier and/or an edible scaffold for producing cultured cells is disclosed. Said microcarrier or scaffold is obtained from natural eggs, by precipitation of egg proteins which serves as microcarriers for cell growth. Said microcarrier or scaffold itself provides for some of the nutrients required for cell cultivation. However, said process involves the use of natural eggs and thus does not circumvent the necessity of animal husbandry. Moreover, since the microcarrier or scaffold is not provided in an encasing, sterility issues may arise.

It was the problem of the present invention to provide a process for the production of foodstuffs from cultured cells that overcomes the above drawbacks.

The above problem has been solved by the present invention.

In detail, the present invention is related to aprocess for cultivating cells or microorganisms, comprising the steps of
a) providing a three-dimensional flexible membrane structure which is permeable to gases and impermeable to liquid and solid components,
b) filling the membrane structure with a composition containing cells or microorganisms,
c) treating the filled membrane structure under cultivation conditions which allow the cells or microorganisms to grow,
characterized in that the membrane structure has a longest dimension of 0.1 cm to 40 cm, preferably 1 to 20 cm.

It has been found that when using a three-dimensional membrane structure with smaller dimensions, there is no need for any movement of the membrane structure during cultivation. A small membrane structure has a large surface area to volume ratio that allows diffusion of a necessary amount of oxygen through the semipermeable membrane structure into any portion of the interior of the membrane structure, without any need for assisting the distribution of oxygen in the membrane structure, for example by any wave-like motion.

The omission of any motion during cultivation allows for the cells in the membrane structure to adhere to surface areas, thus expanding the application possibilities.

Three-dimensional flexible membrane structure that are permeable to gases and impermeable to liquid and solid components are known and commercially available. Reference may be made, for example, to the commercial membranes from Viscofan.

The membrane structure may be made of natural or synthetic materials.

Examples for natural membrane materials are collagen or plant-based or animal-based materials (such as sausage skins). However, also membrane structures based on fungi, algae or bacteria are known. Such membrane structures are typically edible. Examples for synthetic membrane materials are polymers like polyethylene, polypropylene, polyethylene terephthalate, polystyrene, polyamide, polylactic acid, polyvinyl alcohol derivatives such as an ethylene-vinyl alcohol copolymer, or silicones.

The form of the three-dimensional flexible membrane structure is not particularly limited. According to the present invention, cylindrical (sausage-like) membrane structures are preferred.

As stated above, it is important that three-dimensional flexible membrane structure has a dimension that allows diffusion of a necessary amount of oxygen through the semipermeable membrane structure into any portion of the interior of the membrane structure, without any need for assisting the distribution of oxygen in the membrane structure, for example by any wave-like motion. Accordingly, the three-dimensional flexible membrane structure to be used in the present invention has a longest dimension of 0.1 cm to 40 cm, preferably 1 to 20 cm, more preferably 2 to 10 cm.

For the preferred embodiment of a cylindrical (sausage-like) membrane structure, the longest dimension is typically the height of the cylinder, but in some cases the diameter of the cylinder may be larger than the height thereof. According to this embodiment of the present invention, the cylindrical membrane structure preferably has a diameter in the range from 2 to 3 cm.

As stated above, the membrane structure has a large surface area to volume ratio. If the dimensions of the membrane structure are increased, its volume is increased by the power of 3, whereas its surface area is only increased by the power of 2. If cellular material is to be cultivated that requires a high rate of oxygen or vapor supply, the surface area to volume ratio is preferably increased.

According to a preferred embodiment of the present invention, the membrane structure is edible. This results in alternative food products like sausages that can be eaten with their skin. After cultivation, it is not necessary to remove the membrane structure.

According to another preferred embodiment of the present invention, the membrane structure is not edible. This embodiment is used for the manufacture of alternative food products like sausage types where the skin is not eaten, or the manufacture of alternative food products that should have a different form than the form of the membrane structure in which the cultivation takes place. For example, if for the manufacture of an alternative food product in the form of a steak cultivation is carried out in the above described preferred cylindrical membrane structure, the cultivated product has to be removed after cultivation and shaped into the desired steak form.

According to the present invention, the three-dimensional flexible membrane structure is filled with a composition containing cells or microorganisms. According to a preferred embodiment of the present invention, the three-dimensional membrane structure is completely filled with the composition containing cells or microorganisms. In other words, the entire interior of the three-dimensional flexible membrane structure is filled with said composition, with no empty space. This is possible, since the filled three-dimensional flexible membrane structure does not have to be set in motion for generating movement of the composition filled therein. Since the three-dimensional membrane structure to be used in the present invention is flexible, it can expand when the cellular material in its interior increases its volume by growth.

The composition that is filled into the membrane structure contains cells or microorganisms. The term "microorganism" refers to bacteria, single-cell eucaryotic organisms and small plant organisms. Examples are yeast, bacteria, microalgae, fungi, fermented products, mold and the like.

The term "cells" refers here to cells from a multi-cell organism, such as plant cells, fungal cells, or animal cells. Examples are animal cells selected from the group consisting of hepatocytes, myoblasts, osteoblasts, fibroblasts, lipoblasts, odontoblasts, adult neuronal progenitor cells, neural stem cells, multipotent stem cells from subventricular forebrain region, ependymal-derived neural stem cells, hematopoietic stem cells, liver-derived hematopoietic stem, marrow-derived stem cell, adipo-fibroblasts, adipose-derived stem cells, islet-cells producing stem cells, pancreatic-derived pluripotent islet-producing stem cells, mesenchymal stem cells, placenta cells, bone marrow stromal cells, muscle side population cells, bone marrow-derived recycling cells, blood-derived mesenchymal precursor cells, bone-marrow derived side population cells, muscle precursor cells, circulating skeleton stem cells, neural progenitor cells, multipotent adult progenitor cells, mesodermal progenitor cells, spinal cord progenitor cells and spore-like cell, and any combinations thereof. Those animal cells can be cultured or non-cultured cells.

As used herein, the term "animal cells" includes cells derived from fish and seafood and other marine animals.

As used herein, the term "fish and seafood" includes fish, including both freshwater fish and saltwater fish, as well as other marine animals (seafood), such as shrimps.

There is no specific limitation on the kind of cells or microorganisms that can be used. For the manufacture of foodstuffs, cellular material is to be used that is edible and has a high nutrition value.

According to a preferred embodiment of the present invention, the composition containing cells or microorganisms comprises a medium which enables the growth of the cells or microorganisms. Such a medium is typically a culture medium known in the art. Non-limiting exemplary cell culture media that may be purchased from commercial vendors (e.g., Gibco, Sartorius, etc.), or synthesized, include SAFC Excell media, BME (basal Eagle Medium), MEM (minimum Eagle Medium), medium 199, DMEM (Dulbecco's modified Eagle Medium), GMEM (Glasgow modified Eagle medium), DMEM-HamF12, Ham-F12 (Gibco) and Ham-F10 (Gibco), IMDM (Iscove's Modified Dulbecco's medium), MacCoy's 5A medium, RPMI 1640, and GTM3 .

If desired or necessary, such culture media may be supplemented with additional components, such as amino acids, proteins such as growth factors, fats, vitamins, minerals, aroma or flavor substances, edible surfactants, antibiotics, buffering agents, platelet rich plasma (PRP), platelet poor plasma (referred to as plasma), a platelet concentrate, a lysate of red blood cells, a platelet lysate (PL), cytokines, fibers, pectin, alginate, agarose, elastin, chitin, chitosan, fibrin, fibrinogen, polysaccharides, sugars, alginates, collagen, gelatin, polymers or any combination thereof.

The exact ratio of cells/microorganisms and culture medium and optionally additional components depends on the kind of cells/microorganisms and can be readily adjusted by a skilled person.

According to a preferred embodiment of the present invention, the composition containing cells or microorganisms additionally contains fibrous material. As an example, pea fibers which have a fiber content of at least 50 % of their dry weight may be mentioned. The addition of fibrous materials imparts a desired texture to the resulting cultivated product. Preferably, the composition containing cells or microorganisms additionally contains fibrous material in an amount of 0-10 wt.-%, based on the entire amount of the composition.

According to a preferred embodiment of the present invention, the composition containing cells or microorganisms has a structure selected from the group consisting of a gel structure and a scaffold structure. Such as gel structure or scaffold structure resembles the natural growth conditions and results in improved yields and quality of the cultivated product.

A suitable gel structure exhibits a viscoelastic consistency showing viscosity ranges between 1-10000 mPas, as measured with a rotational viscosimeter.

A suitable scaffold structure can be obtained with the method described in WO 2021/032866 A1. In WO 2021/032866 A1, a foamed product by an extrusion process where gas was provided in the extruder. In said foamed product, the dispersed gas fraction and the gas bubble size / size distribution have a quantitative influence on the texture properties (for example, "bite and chewing properties" such as tenderness, hardness, chewability) and make it possible to adapt these sensory properties by adapting the structural parameters to the dispersed gas phase, or by adapting the gas phase, for example increasing the conent of oxygen in the gas phase in order to promote growth of cells or microorganisms. It is assumed that an increase in the gas fraction (up to a matrix-specific critical value) and a reduction in the bubble size (with a constant gas volume fraction) lead to a stiffening of the structure. This leads to an increase in hardness, which is expressed by the maximum force required to start the structural break when cutting/biting, but increases the deformation required for the break and thus influences the chewability. Accordingly, the foaming of the matrix expands the spectrum of tools for adapting, for example, meat-analog bite/chewing properties from chicken to beef and beyond.

Also, the foaming provides for pores in the extrudate into which the cellular material may be inserted.

Said foamed product is made from edible raw materials such as plant protein and starch, and thus is edible.

According to a preferred embodiment of the present invention, the culture medium is sterilized before being used in the process of the invention. Typical sterilization methods such as heat treatment, filtration or irradiation can be used.

Optionally, the three-dimensional flexible membrane structure can also be sterilized or disinfected before filling, preferably using the same sterilization methods as outlined above.

According to the present invention, a culture medium is used that contains all necessary nutrients and ingredients for cell or microorganism growth. This is allows cultivation in a three-dimensional flexible membrane structure which is permeable to gases and impermeable to liquid and solid components, since there is no need for penetration of any liquid or solid growth-supporting components into the interior of the membrane structure.

According to a preferred embodiment of the present invention, the composition containing cells or microorganisms is filled into the three-dimensional flexible membrane structure under aseptic conditions.

Aseptic conditions are known to the skilled person and involves procedures designed to reduce the risk of bacterial, fungal or viral contamination. Preferably, aseptic filling is conducted at ambient temperature conditions such as 20°C, in order to preserve viability of the involved cells or microorganisms.

After filling, the three-dimensional membrane structure is typically closed or sealed, by conventional methods that depend on the nature of the membrane structure. Since the three-dimensional membrane structure is impermeable for liquid or solid contaminants, sterility of the composition in its interior is maintained throughout the process of the present invention.

The filled membrane structure is treated under cultivation conditions which allow the cells or microorganisms to grow. Such cultivation conditions are known to the skilled person.

Preferably, said treatment is carried out in an incubation device. Incubation devices are known to the skilled person. An incubation device (also called incubator) is a device with a sealable chamber that, in said chamber, is able to maintain optimal temperature, humidity and other conditions such as the CO₂ and oxygen content of the atmosphere.

The exact conditions to be set in said treatment step depend on the cells or microorganisms that are to be cultivated. For example, for mammalian cells typically a temperature of 37°C and a relative humidity of 80% or more is suitable for growth.

As stated above, according to the present invention, during said treatment step no movement of the filled membrane structure has to be carried out. Accordingly, no shaking mechanism (or other means of movement) is to be provided in an incubation device to be used according to the present invention.

According to a preferred embodiment of the present invention, a treatment with UV radiation is carried out during step c), i.e. the step of treating the filled membrane structure under cultivation conditions which allow the cells or microorganisms to grow. This may aid in maintaining a desired sterility.

Optionally, during step c), i.e. the step of treating the filled membrane structure under cultivation conditions which allow the cells or microorganisms to grow, the outer surface of the three-dimensional membrane structure can be periodically disinfected with conventional disinfectants such as ethanol.

The treatment step can take, for example several hours up to several days, depending on the desired growth result. Growth can be monitored by conventional methods, such as measurement of the cell density.

One the treatment step (cultivation) is completed, the filled membrane structure is removed from the incubation device for post-processing.

While the process of the present invention can principally be sued for cultivating cells for any purpose (such as the growth of organs or organoids), the present invention is particularly focused on the production of foodstuffs.

Thus, the present invention is also related to a process for the preparation of a food product, comprising the steps of
- cultivating cells or microorganisms in a process according to the invention as described above,
- processing the product thus obtained into the food product, characterized in that the composition containing cells or microorganisms has food quality.

As stated above, the three-dimensional membrane structure may be edible or not edible.

In one preferred embodiment of the present invention, the three-dimensional membrane structure is edible and thus can be part of the final food product (like the skin of certain types of sausages such as a "Bratwurst"). In said embodiment, the product obtained after the treatment step (cultivation) can be used as such as a food product, optionally after a cleaning procedure (e.g. washing with water) or a conventional post-processing step such as smoking. In said embodiment, the step of processing the product thus obtained into the food product may only consist in removing the product from an incubation device and optionally packaging.

In another preferred embodiment of the present invention, the three-dimensional membrane structure is not edible. In said embodiment, the post-processing comprises removing the three-dimensional membrane structure and shaping the cultured composition into the foodstuff.

The membrane structure can be removed by conventional methods such as cutting.

The released fill of the membrane structure, i.e. the composition comprising cultivated cells, can for example be transferred into a mold of a desired shape and cured.

In another preferred embodiment, the post-processing comprises a treatment with a liquid containing ingredients. In other words, the released fill of the membrane structure, i.e. the composition comprising cultivated cells, is brought into contact with ingredient that may provide a desired taste, color or texture. Such ingredients are known and may comprise flavor or aroma substance.

A particularly preferred embodiment involves contacting the released fill of the membrane structure, i.e. the composition comprising cultivated cells, with brine, preferably for 1 to 12 h. Brine is a known salt solution that typically comprises salt (NaCl), spices and flavor.

Said treatment with a liquid containing ingredients can be carried out before or after the released fill is transferred into a mold for shaping.

It is, however, also possible to use the product obtained after the treatment step (cultivation) as such as a food product, and to remove the not edible membrane structure only immediately before consumption (like in the case of other sausage-type products). In said embodiment, the step of processing the product thus obtained into the food product may also only consist in removing the product from an incubation device and optionally packaging.

The process of the present invention allows for a large degree of flexibility in the production of food products, since the dimensions of the three-dimensional membrane structure, the cultivation conditions and the post-processing can be adjusted as desired. Moreover, the process of the present invention allows for adjusting a desired texture of the food product, for example by the use of the specific scaffold material described above or by the addition of fibrous material.

The process of the present invention ensures a high degree of sterility, since after (preferably aseptic) filling into the three-dimensional membrane structure, the composition containing cells or microorganisms is sealed from the environment (apart from a gas exchange), and moreover purification of the outside of the three-dimensional membrane structure is possible throughout the process, without any adverse influence on the sealed filled composition containing cells or microorganisms.

In a preferred embodiment, the process of the present invention is carried out in a large industrial scale. For example, a processing line may be provided where a plurality of three-dimensional membrane structure are transported between various processing stations, such as a filling station for filling the three-dimensional membrane structure with a composition containing cells or microorganisms, a treatment station (such as an incubator) where cultivation takes place, an a post-processing station.

Preferably, said treatment station is dimensioned such that a plurality of three-dimensional membrane structures can be subjected simultaneously to cultivation.

## Claims

1. A process for cultivating cells or microorganisms, comprising the steps of
a) providing a three-dimensional flexible membrane structure which is permeable to gases and impermeable to liquid and solid components,
b) filling the membrane structure with a composition containing cells or microorganisms,
c) treating the filled membrane structure under cultivation conditions which allow the cells or microorganisms to grow,
**characterized in that** the membrane structure has a longest dimension of 0.1 cm to 40 cm, preferably 1 to 20 cm.

2. The process according to claim 1, **characterized in that** the composition containing cells or microorganisms comprises a medium which enables the growth of the cells or microorganisms .

3. The process according to claim 1 or 2, **characterized in that** step b) is carried out under aseptic conditions.

4. The process according to one of the preceding claims, **characterized in that** in step b) the three-dimensional membrane structure is completely filled with the composition containing cells or microorganisms.

5. The process according to one of the preceding claims, **characterized in that** the three-dimensional membrane structure is closed or sealed after filling in step b).

6. The process according to one of the preceding claims, **characterized in that** step c) is carried out in an incubation device.

7. The process according to one of the preceding claims, **characterized in that** a treatment with UV radiation is carried out during step c).

8. The process according to any one of the preceding claims, **characterized in that** the composition containing cells or microorganisms has a structure selected from the group consisting of a gel structure and a scaffold structure.

9. The process according to one of the preceding claims, **characterized in that** the composition containing cells or microorganisms additionally contains fibrous material.

10. The process according to any one of the preceding claims, **characterized in that** the membrane structure is edible.

11. The process according to one of the preceding claims, **characterized in that** the membrane structure is not edible.

12. A process for the preparation of a food product, comprising the steps of
- cultivating cells or microorganisms in a process according to any one of claims 1 to 11,
- processing the product thus obtained into the food product,
**characterized in that** the composition containing cells or microorganisms has food quality.

13. The process according to claim 12, **characterized in that** the post-processing comprises removing the three-dimensional membrane structure and shaping the cultured composition into the foodstuff.

14. The process according to claim 13, **characterized in that** the post-processing comprises a treatment with a liquid containing ingredients.
